# EUROPEAN PATENT SPECIFICATION

(11) **EP 3 986 269 B1**
(45) Date of publication and mention of the grant of the patent: **06.08.2025**
(21) Application number: 20827729.3
(22) Date of filing: 18.06.2020
(51) Int. Cl.: A61B 5/05, A61B 5/00

(54) **FAT BURNING MONITORING**
ÜBERWACHUNG DER FETTVERBRENNUNG
SURVEILLANCE DE LA COMBUSTION DE GRAISSE

(30) Priority: 21.06.2019 US 201962864827 P
(43) Date of publication of application: 27.04.2022
(73) Proprietor: 1625986 Ontario Limited, Mount Brydges, Ontario N0L 1W0 (CA)
(72) Inventor: TAYLOR, Geoffrey Locke, Winnipeg, Manitoba R3Y 1G4 (CA)
(74) Representative: Berggren Oy
(86) International application number: PCT/CA2020/050844
(87) International publication number: WO 2020/252580

(56) References cited:
- WO-A1-2018/085944
- WO-A1-2020/117918
- US-A1- 2013 245 388
- US-A1- 2015 044 098
- US-B2- 8 652 042
- THORNTON SIMON N.: "Increased Hydration Can Be Associated with Weight Loss", FRONTIERS IN NUTRITION, vol. 3, 10 June 2016 (2016-06-10), XP093044179, DOI: 10.3389/fnut.2016.00018

## Description

### BACKGROUND OF THE INVENTION

Currently just over a third of men are overweight or obese, and nearly 40% of women are (Ng M, Fleming T, Robinson M et al. Global, regional and national prevalence of overweight and obesity in children and adults during 1980-2013: A systematic analysis for the Global Burden of Disease Study 2013. Lancet 2014; 384 (9945); 766-81).

Once considered a high-income country problem, overweight and obesity are now on the rise in low- and middle- income countries, particularly in urban settings. These countries are now facing a "double burden" of disease. While they continue to deal with the problems of infectious disease and under-nutrition, they are experiencing a rapid upsurge in non-communicable disease risk factors such as obesity and overweight. In developing countries with emerging economies (classified by the World Bank as lower- and middle-income countries) the rate of increase of childhood overweight and obesity has been more than 30% higher than that of the developed countries, and overweight and obesity are linked to more deaths worldwide than underweight. For example, 65% of the world's population live in countries where overweight and obesity kill more people than underweight (this includes all high-income and most middle-income countries).

The causes of obesity are as varied as the people it affects. At its most basic, of course, obesity results when an energy imbalance occurs due to a difference in calories consumed and calories expended. The body stores these excess calories as body fat, and over time the extra pounds add up. Eat fewer calories than the body burns, weight goes down. This equation can be deceptively simple, though, because it does not account for the multitude of factors that affect what we eat, how much we exercise, and how our bodies process all this energy.

Use of sugar substitutes may add to obesity in that they elicit an increase in insulin (like a normal sugar would do) but in an amount that exceeds the actual glucose levels and this causes the body to store more of the available glucose as fat.

The health consequences associated with overweight and obesity are numerous and at the rate they are progressing the economic burdens will soon begin to cripple many countries. For example, 44% is attributed to the diabetes burden, 23% of the heart disease burden and between 7% and 41% of certain cancer burdens are attributable to overweight and obesity.

The following are the major risks: breathing disorders (e.g., sleep apnea, chronic obstructive pulmonary disease); certain types of cancers (e.g., prostate and bowel cancer in men, breast and uterine cancer in women); coronary artery (heart) disease; depression; diabetes; gallbladder or liver disease; gastro esophageal reflux disease (GERO); high blood pressure; high cholesterol; joint disease (e.g., osteoarthritis); stroke.

Aside from the medical complications, obesity is also linked to psychosocial problems such as low self-esteem, discrimination, difficulty finding employment, and reduced quality of life, thus leading to depression, eating disorders and crash diets.

There is also physiological phenomena wherein obesity triggers a reduction in hormones (for example leptin which is produced by fat cells) that would normally suppress the appetite and body fat plays an important role in the development of obesity-related conditions such as heart disease, stroke and some forms of arthritis.

There are hundreds of diets, diet aids, supplements and vitamins, exercise and lifestyle experts located all over the world that are trying to help eradicate this global epidemic. However, what seems to be such a simple concept of eat less and exercise more will equate to weigh less and be healthy does not seem to be achievable for the general masses of the world.

Weight obesity and its causes have, in many ways, become woven into the fabric of our society. To successfully disentangle them will take a multifaceted approach that not only gives individuals the skills to make healthier choices but also sets in place policy and infrastructure that support those choices.

Fat loss has been directly corelated to acetone (in this case breath acetone) in research done by Joseph C. Anderson "Measuring breath acetone for monitoring fat loss" published in the Obesity Research Journal in Nov 2015.

Although the health risks are modifiable or preventable there are very little efforts in progress in most of the developing world to control them. We believe there needs to be a simple device to measure and achieve weight management to reduce the economic burden of the disease worldwide.

Cells break down or burn carbohydrates, amino acids and fats to generate ATP, the universal energy currency of cells. In mammals, adipose tissue is the major storage site for fat. Ketones are produced when the body burns fats stored in adipose tissue for energy or fuel. They are also produced when there is not enough insulin to help the body use sugar for energy. Without enough insulin, glucose builds up in the blood. Normally, the body gets the energy it needs from carbohydrate in the diet. Stored fat can only be broken down by turning it into ketones and that only happens if the diet does not contain enough carbohydrates to supply the body with sugar (glucose) for energy or if your body can't use blood sugar (glucose) properly.

The excretion of ketone bodies in urine is very low and undetectable by routine urine tests (Rothera's test). However, when the rate of synthesis of ketone bodies exceeds the rate of utilization, their concentration in blood increases; this is known as ketonemia. This is followed by ketonuria - excretion of ketone bodies in urine. The overall picture of ketonemia and ketonuria is commonly referred as ketosis. Being in a state of ketosis is associated with the fat burning metabolic process (Attia, P., "Description of Ketosis"). Smell of acetone in breath is a common feature in ketosis. The occurrence of high levels of ketone bodies in the blood during starvation, a low carbohydrate diet, prolonged heavy exercise and uncontrolled type 1 diabetes mellitus is known as ketosis, and in its extreme form in out-of-control type 1 diabetes mellitus, as ketoacidosis.

When a type 1 diabetic suffers a biological stress event (sepsis, heart attack, infection) or fails to administer enough insulin they may suffer the pathological condition ketoacidosis. Liver cells metabolise fatty acids into ketones in an attempt to supply energy to peripheral cells which are unable to transport glucose in the absence of insulin. The resulting very high levels of blood glucose and ketone bodies lower the pH of the blood and trigger the kidneys to attempt to excrete the glucose and ketones. Osmotic diuresis of glucose will cause further removal of water and electrolytes from the blood resulting in potentially fatal dehydration, tachycardia and hypotension.

### The Ketone Bodies

Ketone body metabolism is highly regulated and circulating levels of ketone bodies are determined by their rates of production and utilization. Ketone production and use is aggressively suppressed by glucose. As glucose stores become depleted the body's metabolism switched to reducing fat to produce ketone bodies. In humans, basal serum concentration of β-hydroxybutyrate is in the low micromolar range, gradually increasing to 1-2 mM after 2 days of fasting and 6-8 mM with prolonged starvation. Ninety minutes of intense exercise can also cause β-hydroxybutyrate concentration to increase to 1 - 2mM while uncontrolled diabetics can have β-hydroxybutyrate concentrations of 25mM.

Individuals who follow a low-carbohydrate diet will also develop ketosis, sometimes called nutritional ketosis, but the level of ketone body concentrations are on the order of 0.5-5 mM whereas the pathological ketoacidosis is 15-25 mM.

Type 1 diabetic patients lack insulin and the build up of ketones, leads to ketoacidosis from low serum insulin levels.

### Measurement of Ketone in Blood

A blood test analyzed by a laboratory is the most accurate method of measuring ketones. However, laboratories' tests are not practical for people following a diet, or for prompt testing.

"In-house", current methods for measuring levels of ketones include urine strips, or invasive devices (pricking of your finger). However, while urine strips measure excess ketones excreted in urine and a blood test measures ketones available in blood, neither measures the immediate use of ketones.

### Measurement of Ketones in Breath

U.S. Pat. No. 8,871,521 ("US ·521") discloses a breath ketone detector. Although non-invasive, this device has many disadvantages. Diabetic ketoacidosis is characterized by the accumulation of ketone bodies in blood; however, this device allegedly measures ketone bodies in breath and, therefore, it represents an indirect measure of blood ketones. The device is disposable, i.e. designed for one-time use, which means that a user in need, must always make sure to have an unused device available. The device has an expiry date at ambient, which means that a user in need cannot keep too large a stock of devices. The operation of the device may not be suitable for most users; indeed, according to US '521 a user must "take a deep breath and blow for 30 seconds through the end of the tube designated by arrow. Blow very hard. Exhale through the tube, do not inhale."

US Pat. No. 9,456,749 discloses a portable device having an integrated chemical sensor sensitive to ketones within a breath sample. This device is incapable of registering electric conductivity of the skin.

US2009/0054799 discloses a sensor with a breath delivery system with the breath sensor capable of detecting an analyte in the breath, including acetone. Breath activates the breath sensor resulting in an electrical signal that is transmitted to portable electronic device.

US2016/0331272 discloses a portable device for measuring acetone in breath using a nanoparticle-based sensor. This document teaches that electrical aspects of the breath measurement devices pose risks as electrostatic discharges can occur that may impact the device.

We have found that the highest concentrations of acetone are found in the very last of the exhaled breath and this can lead to large variations in breath acetone readings.

Other prior art documents include: US2016/0371590, WO2016/192941, US8,453,601, and US2015/0073290.

None of the devices of the prior art measure surface conductivity or conductivity due to breath condensates (neither of which is a gas) nor a correlation between the surface or breath conductivity and ketosis.

As can be seen, the range of technologies suitable for non-invasive, repeated, in-vivo measurement of blood ketones is rather limited. A suitable technology needs to be sensitive to blood chemistry, ideally not require the addition of reagents in order to make the measurement and present no hazard to the patient or operator. Technologies that potentially meet these requirements include UV-Vis, mid infrared, near infrared, Raman and Terahertz (far infrared) spectroscopy. However, ketone bodies are small molecules, and it is not possible to get a reliable measurement of ketone bodies across an intact skin.

Cardiovascular machines such as treadmills, stationary bikes, elliptical trainers, stair steppers, to name a few, come with a variety of widgets that let the user measure his/her heart rate and calculate the approximate amounts of calories being burnt. However, neither heart rate nor calories burnt may represent that the person is burning fat. Regarding calories, exercise requires energy, and this energy is measured using calories. The source for energy may come from either fat or muscle glycogen. As the body adapts to each exercise, it becomes more efficient in its use of calories, therefore burning less. The amount of muscle also has an impact on calories burned. Because muscle is metabolically active - more muscle means more calories being burned. The so called "fat burning zone" may be calculated by using the resting heart rate (RHR), the maximum heart rate (MHR), and the heart rate reserve (HRR) as follows: Fat burning range = RHR + HRR x 0.5. However, this relationship may depend on the level of fitness of the person and his/her age. Therefore, a cardiovascular machine that more accurately measures when a person is burning fat, is needed. None of the currently used devices for measuring ketone levels may be adapted for exercise machines.

Presented herein are devices and methods that let users see firsthand what the treatments, foods or exercises that are available to them are doing to their own personal metabolism and the burning of fat in their bodies, and how they can take control and monitor their own personal success by self-empowerment. It should be understood that the applicants are not advocating or intending to enable unsafe, unsupervised extreme dieting, but rather taking the view that the diets have to be properly constituted and that people following these diets should be under medical supervision.

We have found that while the available ketones can be measured using a blood test and excess ketones can be measured using a urine strip, only continuous measuring of the presence and concentrations of acetone, is indicative of the periodic production of ketones from available fats.

We also are aware that exogenous ketones such as MCT oils or other foods rich in ketones can be ingested and give high ketone readings. High readings in this case are not indicative of fat burning. They are only indicative of available ketones if tested for in the blood or surplus ketones if tested for in the urine.
US 2013/0245388 A1 describes an apparatus for monitoring a condition of a tissue based on a measurement of an electrical property of the tissue. In an example, the electrical property of the tissue is performed using an apparatus disposed above the tissue, where the apparatus includes at least two conductive structures, each having a non-linear configuration, where the at least two conductive structures are disposed substantially parallel to each other. In another example, the electrical property of the tissue is performed using an apparatus disposed above the tissue, where the apparatus includes at least one inductor structure.
US 2015/0044098 A1 briefly describes a capacitance method for measuring skin moisture content in humans. The method treats the water content in the skin as a dielectric material. Thus, for a capacitance measuring device, the increase in capacitance is proportional to the quantity of water in the skin. The exact type of transducer used for capacitance measuring devices in relation to skin moisture is called an "Interdigital Capacitor."
Thornton. Simon N: "Increased Hydration Can Be Associated With Weight Loss". Frontiers in Nutrition, vol. 3. 10 June 2016 describes a hypothesis that increased hydration leads to body weight loss, mainly through a decrease in feeding, and a loss of fat, through Increased lipolysis. The hypothesis derives from a broader association between chronic hypohydration (extracellular dehydration) and raised levels of the hormone angiotensin II (AngII) associated with many chronic diseases, such as obesity, diabetes, cancer, and cardiovascular disease.
WO 2018/085944 describes an apparatus and method of monitoring fat burning in a subject non-invasively by measuring electrical conductivity or resistance on the skin.
US 8652042 B2 describes a human skin moisture measuring device having an interdigitated resistive sensor formed on a plate-like transparent support is described, with a face designed to be placed into contact with the skin to be subjected to the moisture degree measurement. The device further comprises image sensing means turned towards the opposite face of the support and a lighting device arranged sideways relative to the support to direct the light radiation in the support at a prefixed angle. Means for processing the signal coming from the interdigitated resistive sensor and from the image sensing means give as output a value of the measured moisture degree that is normalized with respect to the actual surface of contact between skin and sensor.

### SUMMARY OF THE INVENTION

According to an aspect of the invention, there is provided a device comprising:
(i) an interdigitated portion having two opposing ends and configured for contacting skin of a subject and measuring skin capacitance;
(ii) a ketosis signal, and
(iii) a capacitance meter;
wherein the ketosis signal is activated when the skin capacitance measurement is in the nano farad range.

According to another aspect of the invention, there is provided a method for non-invasively detecting if a subject is in ketosis comprising:
providing a device comprising:
an interdigitated portion for measuring capacitance of skin of the subject, said interdigitated portion having at least two separate electrical contacts; and
a ketosis signal responsive to one end of the interdigitated portion, said ketosis signal being activated when a skin capacitance measurement of the subject increases compared to a comparative capacitance value of the subject,
placing the device on a body of the subject such that said two electrical contacts of said interdigitated portion are contacting the skin of the subject; and
said device measuring the capacitance of the skin of the subject;
wherein said device activates the ketosis signal when the skin capacitance measurement is different from the comparative capacitance measurement of the subject.

### BRIEF DESCRIPTION OF THE DRAWINGS

Figure 1. The 3 "Ketone bodies". D-p-hydroxybutyric acid (POHB), although properly not a ketone, is interchangeable with acetoacetic acid via POHB dehydrogenase. Its equilibrium is a function of the NADH/NAD⁺ ratio. Acetoacetic acid is slowly and irreversibly decarboxylated to acetone which is mainly lost to the environment through skin, lungs and urine.
Figure 2. Schematic diagram of a circuit diagram of the capacitance meter of the present invention.
Figure 3. Schematic diagram showing three differently spaced interdigitated contact sensors.

### DESCRIPTION OF THE PREFERRED EMBODIMENTS

### Definitions

Unless defined otherwise, all technical and scientific terms used herein have the same meaning as commonly understood by one of ordinary skill in the art to which this invention belongs. Also, unless indicated otherwise, except within the claims, the use of "or" includes "and" and vice versa. Non-limiting terms are not to be constmed as limiting unless expressly stated or the context clearly indicates otherwise (for example "including", "having" and "comprising" typically indicate "including without limitation"). Singular forms including in the claims such as "a", "an" and "the" include the plural reference unless expressly stated otherwise. In order to aid in the understanding and preparation of the within invention, the following illustrative, non-limiting, examples are provided.

"Ketone body" refers to molecules that are produced by the liver from fatty acids during periods of low food intake (fasting) or carbohydrate restriction for cells of the body to use as energy instead of glucose. Examples of ketone bodies include acetone, acetoacetic acid, and beta-hydroxybutyric acid.

When fat is being used as the primary source of cellular energy ketones are produced and in this document, the term "fat-burning molecules" refers to one or more molecules whose presence or absence in blood or interstitial fluid or whose presence in blood at or above a normal threshold is indicative of stored fats being used. Non-limiting examples of fat-burning molecules include acetone, acetoacetic acid, and beta-hydroxybutyric acid, insulin, glucagon, free fatty acids and glycerol.

As used herein, "skin capacitance measurement" means that the skin capacitance is measured either at the surface of the skin or at a given depth below the surface of the skin, as discussed herein.

Subject includes any mammal, including humans.

### Overview

The present invention relates to methods and devices for the non-invasive monitoring of fat burning in a subject.

Acetone is a normal metabolite and its presence in breath levels ranges from a relatively high 0.5ppmv for healthy individuals to hundreds of ppmv for critically ill, ketoacidotic diabetics. Acetone readily equilibrates throughout the body and can also be detected emanating from the skin. Acetone emitted from the skin correlate directly with the breakdown of beta-hydroxybutyrate into acetoacetic acid and the subsequent decarboxylation to acetone.

This has been confirmed by using gas chromatography.

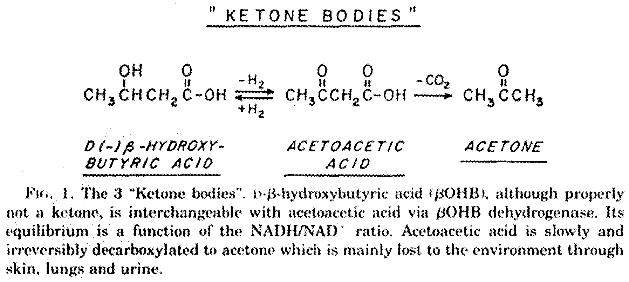

Skin acetone concentrations of persons with diabetes (about 188±17 ppb) are significantly higher than normal subjects (about 87±10 ppb). Skin acetone concentrations are related to blood beta-hydroxybutyrate, blood glucose and HbAlc in patients with diabetes. Skin acetone concentration is high (approx. 940 ppb) in a patient with diabetic ketoacidosis and drops to approximately 80 ppb after insulin therapy. These compounds are shown in Figure 1.

Skin acetone is herein used as a screening test for ketosis and for diabetic control and for ketone production in diabetes and other ketogenic conditions.

In one embodiment, the present invention is an apparatus or device for indicating or monitoring fat burning or ketone production by measuring skin capacitance.

Capacitance of acetone is about 40 picofarads. The capacitance of sweat is about 40 micro farads. Given that acetone is hydrophobic, it further helps separate the influence of salts naturally occurring on the skin. The presence of acetone also changes the mechanical properties of the skin.

Specifically, described herein is a method of measuring skin capacitance wherein an increase in acetone production leads to an increase in capacitance which in turn is indicative of ketosis. As will be appreciated by one of skill in the art, while capacitance values will vary between individuals, in some exemplary examples, capacitance when in ketosis may be about for example 10 nano farad and while not in ketosis, capacitance of the skin may be for example about 100 pico farad.

As discussed herein, in some embodiments, the skin capacitance measurement is compared to a baseline skin capacitance value taken when the subject is not in ketosis, that is, the baseline skin capacitance value may represent the "normal" skin capacitance measurement or value of the subject. The fact that the individual is not in ketosis at the time of this reading can be validated by a number of methods, for example, with a urine keto strip (indicating that excess ketones are being excreted in urine) or a blood test (indicating that there are ketones available in the blood) or with an acetone breath analyzer (indicating that fat is being burned and that ketones are being consumed). As discussed herein, the user could also breathe on the interdigitated sensor portion of the device as a measure of breath acetone. As will be appreciated by one of skill in the art, if necessary, the subject can confirm the validity of the readings from the device by repeating the confirmatory test when they are in ketosis, for example, when the device indicates they are in ketosis, that is, when the skin capacitance has changed, as discussed herein.

Alternatively, in other embodiments, the skin capacitance measurement is compared to a comparative skin capacitance value. As discussed herein, the skin capacitance value may be indicative of a desirable degree or level of fat burning or ketosis.

In some embodiments, the skin capacitance measurement, the baseline capacitance value and the comparative capacitance value are all taken at the same skin depth, for example, at the skin surface.

As will be apparent to one of skill in the art, the farad (symbol: F) is the derived unit of electric capacitance.

Previously, the inventor developed a fat-burning monitoring device that measured skin conductance/resistance. This was based on the hypothesis that because acetone is 1000 times more conductive than normal (salty) skin, the conductance of the skin surface would be indicative of the acetone build up on the skin. However, it was discovered that the skin and underlying tissue act effectively like a leaky capacitor. Furthermore, while the expected resistance changes on the skin surface were present, under certain conditions, these readings can be influenced by evaporation and therefore can be influenced by the tightness of the sensor to the skin. For example, in some instances, when the device loses contact with the skin, the conductance reading goes to zero.

It was also found that contact force can have an effect on the conductance reading. Specifically, it was found that for greatest accuracy, it was important to maintain a consistent contact force during measurements, as it was found that the greater the force applied to the sensors, the greater the conductance measured. As will be apparent to one of skill in the art, maintaining constant force and contact is difficult at best when exercising.

Yet further, conductance, measured using an applied DC voltage, really measures only one thing: the surface conductance, meaning that it is not possible to measure conductance at different depths. As discussed herein, measurements of capacitance at different depths provides valuable information regarding changes in fat burning in real time, that is, trends in ketosis, without the need for comparison to a baseline capacitance value or a comparison capacitance value.

As discussed above, it was found that conductance/resistance is variable due to sweat; however, capacitance is much less variable. Specifically, it was found that capacitance remains in a measurable range when the user is in or out of ketosis and the extremes could be measured, as discussed herein. Furthermore, using different frequencies, we can measure capacitance below the surface of the skin which is subject to even less variability.

Surprisingly, given our experiences with conductance/resistance, slight losses of contact do not seem to impact capacitance. Furthermore, the overall variations in capacitance were stable and of the order of two orders of magnitude. While not wishing to be bound to a particular theory or hypothesis, it is believed that because air has a measurable capacitance, continuity of reading was not lost if the sensor contact force changed or the sensor momentarily lost contact. It may also be that the contact sensor retains its capacitance for a period of time after losing contact due to its surface wetness.

As will be appreciated by one of skill in the art, capacitance was initially considered unsuitable for measuring fat burning because it was expected that as the acetone built up on the skin's surface, the conductance would increase (which it did) but that the capacitance would drop (it did not). Surprisingly, capacitance was not the inverse of the conductance as expected but in fact when conductance increased, so did the capacitance. While not wishing to be bound to a particular theory or hypothesis, it is believed that this is due to the fact that interstitial fluid is composed primarily of electrolyte salts that have a capacitance in the range of 40,000,000 pico farads whereas acetone has a capacitance in the order of 40 picofarads so as the acetone built up, the capacitance increased dramatically and in perfect correlation to the breath acetone levels. Furthermore, the mechanical properties of the skin changed as acetone levels built up in the interstitial fluid and that seemed to vary the capacitance dramatically as well. While not wishing to be bound to a particular theory or hypothesis, we suspect that the acetone changed the surface acoustic wave properties and also the sub-surface malleability of the subcutaneous tissue such that the sound waves influenced the way the current flowed through the tissue and that this changed its capacitance.

As discussed above, it was initially anticipated that conductance could be measured below the surface of the skin and that this could be used to provide greater information on the degree of ketosis or fat burning, for example, by profiling the acetone gradient that formed. However, as discussed above, with conductance, we could not measure under the skin, that is, below the skin surface.

While this was not possible when measuring conductance, it was possible when measuring capacitance. As discussed herein, in some embodiments, skin capacitance measurements are taken at different depths. In some embodiments, the depth is a function of both frequency and the spacing of the interdigitated contacts. For example, closer spacing between the interdigitated contacts are suitable for reading surface conditions, that is, for measuring skin surface capacitance, while greater spacing, that is, increased distance between interdigitated contacts, measures deeper below the skin surface. Put another way, low frequency reads deeper and high frequency reads shallower relative to the skin surface. It is noted that suitable spacing for measuring capacitance at a particular depth and/or for generating a particular frequency will be readily apparent to one of skill in the art and/or can be determined by routine experimentation. For example, in some embodiments, the frequencies may be from about 0 Hz or DC to about 100 kHz. In other embodiments, the device may be arranged to scan through two or more frequencies, for example, through a plurality of frequencies.

As will be appreciated by one of skill in the art, the different frequencies give information at different depths relative to the skin surface. For example, lower frequencies provide a reading that is at a greater depth relative to the skin surface than higher frequencies, which will provide a reading of capacitance at a point that is closer to the skin surface. This is true for all the contact spaces and different for each of the separation distances, meaning that large data sets of capacitance measurements at different skin depths can be attained, as discussed herein.

As discussed herein, the shape of the curves, at each frequency and at any given spacing of the interdigitated contacts, varies as a function of the mechanical and dielectric properties of the tissue through which the signal is being transmitted. These properties vary as the different tissues absorb and change as fluids (blood, interstitial fluids, and other fluids) are moving in and out of the individual cells or flowing through/around the tissues.

As will be appreciated by one of skill in the art, the spaces between frequencies is unlimited meaning that any suitable number of frequencies, differing by any suitable range, may be used. However, in some embodiments, 10 kHz spaces between sampling is used. For example, in some embodiments, each of the four space is sampled at each of 10 kHz frequency steps between 10 kHz and 100 kHz, which can be used to construct a 3D set of data which can be used to construct an image of the acetone concentrations in the volume of tissue below the contacts of the device.

Accordingly, in one aspect of the invention, the device is arranged to scan through two or more frequencies from between 0 Hz or DC to about 100 kHz. In some embodiments of the invention, the device scans two or more frequencies selected from the group consisting of about 0 Hz (DC), about 10kHz, about 20 kHz, about 30 kHz, about 40 kHz, about 50 kHz, about 60 kHz, about 70 kHz, about 80 kHz, about 90 kHz and about 100 kHz.

As will be appreciated by one of skill in the art, a device with such a scanning mode can be prepared by a variety of different means, using means known to those of skill in the art.

For example, in one embodiment of the invention, each set of interdigitated contacts is subjected to a respective current of a given frequency, said respective current having a frequency from 0 kHz (DC) to 100 kHz. As will be appreciated by one of skill in the art, the source contacts can be used as the pickup or other contacts can be used to detect the signal as it has been modified by the tissues.

Alternatively, this could be done using an array of point sources where the signals are injected and read from the different points.

As will be known by those of skill in the art, skin capacitance can be affected by a number of factors, for example, but by no means limited to skin tightness, skin dryness, psoriasis, and skin penetration of cosmetics, including sun screens, lotions and the like.

In some embodiments of the invention, skin surface resistance is measured in combination with the skin capacitance measurements. As will be appreciated by one of skill in the art, measuring resistance at DC voltages gives different mechanical properties information. Specifically, DC typically corresponds to larger bodies whereas the higher frequencies tend to excite and be influenced by smaller bodies (at their related vibrational harmonics).

For example, resistance can be used in combination with capacitance readings to remove the influence of sweat on capacitance readings since sweat changes the surface resistance much more than it changes the surface capacitance.

Specifically, resistance is generally low when capacitance is high and vise versa. Resistance measures the electron flow over a long period of time, in one direction and can be compared to the electron flow in the opposite direction, for example, either against the blood flow or the interstitial fluid flows. This can be used to infer some depth information by comparing the current flow between the closely spaced and the wider spaced contacts (closer spaces being shallower), as described herein.

In another embodiment of the invention, there is provided a method for training a machine learning algorithm or an artificial intelligence to detect electrical changes associated with physiological events comprising:
a step S1, obtaining electrical input data and physiological input data from a subject, to be transmitted, as inputs, to the machine learning algorithm;
a step S2, applying one or more data analysis methods to detect patterns and extract features within the electrical input data and the physiological input data;
a step S3, subjecting the detected patterns and extracted features from the electrical input data and the physiological input data to multivariate analysis to detect interdependence between them; and
a step S4, reporting electrical changes associated with physiological events.

In some embodiments, electrical input data includes but is not necessarily limited to skin resistance, skin surface capacitance and skin subsurface capacitance. In some embodiments of the invention, the skin subsurface capacitance comprises capacitance readings taken at more than one depth, for example, at more than one frequency, as discussed herein.

In some embodiments, the device is arranged to scan through two or more frequencies from between 0 Hz or DC to about 100 kHz. In some embodiments of the invention, the device scans two or more frequencies selected from the group consisting of about 0 Hz (DC), about 10kHz, about 20 kHz, about 30 kHz, about 40 kHz, about 50 kHz, about 60 kHz, about 70 kHz, about 80 kHz, about 90 kHz and about 100 kHz.

As will be appreciated by one of skill in the art, the physiological input data comprises physiological parameters indicative of a specific physiological state.

In some embodiments of the invention, the correlation of electrical changes and physiological events allows for prediction of physiological events to be made from electrical readings, as discussed herein.

For example, physiological data may include but is by no means limited to electrodermal data, for example, breathing rate data, blood pressure data, oxygenation rate data, cardiac activity data, heart rate data, pulse oximetry data, respiration data, temperature data, moisture data, humidity data, location data, and oxygen saturation data.

In some embodiments, at least some of the physiological data is reported by one or more sensors known in the art for measuring and reporting such data. It is of note that in some embodiments, at least some of the physiological data may be entered manually.

As will be appreciated by one of skill in the art, the physiological data may be reported to the fat burning measurement device as described herein or both the physiological data and the electrical data may be reported for analysis, as described herein. This data may be reported for analysis in real time or may be stored for download at a later time.

As will be appreciated by one of skill in the art, the electrical data and the physiological data may be obtained simultaneously and/or may be time stamped. Specifically, it is expected that in some cases, a physiological state or a change in a physiological state may be preceded by a change in skin surface resistance or skin capacitance, for example, skin capacitance at a specific skin depth, as discussed herein. Accordingly, these changes in capacitance and/or resistance may be predictive of certain physiological states or events.

For example, temperature date may include both body temperature, to correlate with physiological states such as fever, excitation and the like, and air temperature, for example, to look at the influence of body temperature and/or ambient temperature and/or the difference therebetween on perspiration rates and depth profiling of salinity concentrations.

In some embodiments of the invention, there is a computer system implementing a machine learning algorithm.

The machine learning algorithm may comprise, for example, an artificial neural network or a support vector machine. The machine learning algorithm may also comprise one or more data analysis methods selected from the group consisting of but by no means limited to, one or more data analysis methods selected from the group consisting of: non-linear regression, linear regression, logistic regression, a decision tree, a support vector machine, a naïve bayes, K-nearest neighbors, K-means, random forest, dimensionality reduction, neural network, gradient boosting and/or adaboost MLA.

In some embodiments, the MLA may be re-trained or further trained by the computer system based on data collected from one or more subjects and/or from sensors and/or input devices and/or the fat burning monitor device as described herein.

In some embodiments, the device includes a memory module for storing information regarding the capacitance measurements. While this may include historical data on capacitance readings or measurements over time, this may also include information specific for a given user, for example, the baseline capacitance value, indicative of when the user is not in ketosis; one or more comparative capacitance values, indicative of when the user is in a particular degree or level of ketosis or fat burning; and/or frequencies for accurate depth capacitance measurements, as discussed herein.

The present invention relates, in one embodiment, to an apparatus and method for continuously monitoring fat-burning in a subject.

In one embodiment, the present invention relates to an apparatus for monitoring fat burning in a subject non-invasively, the apparatus having means for measuring an electric capacitance of the skin of a subject, and at least one out-put signal configured to indicate when the electrical capacitance of the skin is indicative of the subject burning fat, that is, being in ketosis. As will be appreciated by one of skill in the art, the signal may indicate that the subject is in ketosis by being active or by being inactive. In some embodiments, the signal may be an electronic signal that is transmitted to a display of the device or to another device, such as, for example, a smart phone or a remote database, for example, a cloud-based database. For example, a change in capacitance associated with going into ketosis could be a signal to another device directly.

In one embodiment, the apparatus comprises a capacitance meter for monitoring fat burning in a subject. The apparatus, in one embodiment, comprises a capacitance meter including: (i) an interdigitated portion having two opposing ends and configured for contacting skin of a subject and measuring surface capacitance and/or subsurface capacitance of the subject's skin; (ii) a ketosis signal that reflects or is activated when the capacitance of the skin is increased compared to a baseline capacitance measurement, and is indicative of the subject burning fat, the ketosis signal being connected to or operably linked to one end of the interdigitated portion and (iii) a capacitance meter.

In some embodiments, the baseline capacitance measurement is taken when the user is not in ketosis and is indicative of the user not burning fat. As discussed herein, this can be verified for example by the absence of breath ketones or by other means known in the art, as discussed herein.

In other embodiments, the baseline capacitance measurement may be an initial reading taken as a comparative capacitance measurement or value, that is, a measurement for comparison to indicate if the degree or level of fat burning is changing over time. For example, when the capacitance of the skin is reduced compared to a previous capacitance measurement, this is indicative of an increase in fat burning or an increase in the level of ketosis of the user.

As discussed herein, skin capacitance measurements can vary from person to person. However, typically, an individual who is not in ketosis, that is, who is not actively burning fat, will have a skin capacitance in the pico farad range, for example, about 100 pico farad whereas an individual who is in ketosis, that is, who is actively burning fat, may have a skin capacitance that is in the nano farad range, for example, about 10 nano farad. As will be apparent to one of skill in the art, the fat burning skin capacitance and the non-fat burning skin capacitance vary significantly. Furthermore, because the device can be used to take a baseline capacitance measurement, which may be a measurement of skin capacitance taken when the individual is not in ketosis or may be a comparative skin capacitance measurement taken previously, the device can measure changes in the user's skin capacitance and does not necessarily rely on absolute values.

Furthermore, in some embodiments, the ketosis signal may be a digital signal or may be a segmented or progressive signal that is arranged to provide greater detail on the skin capacitance. For example, the ketosis signal may provide a digital readout of the actual or current skin capacitance or may provide a digital readout of the difference between the actual or current skin capacitance and the baseline or comparative skin capacitance measurement either to the device or to a remote device such as for example a smart phone. Alternatively, the ketosis signal may provide a visual representation of these values, that is, a "stronger" signal, for example, activation of more segments of the signal, which is indicative of a greater degree or level of ketosis being attained by the user.

In some embodiments of the invention, there is provided a method for non-invasively detecting if a subject is in ketosis comprising:
providing a device comprising:
   an interdigitated portion for measuring capacitance of skin of the subject, said interdigitated portion having at least two separate electrical contacts;
   a power source; and
   a ketosis signal connected or operably linked to one end of the interdigitated portion, said ketosis signal being activated when the capacitance of the skin of the subject increases compared to a comparative capacitance value of the skin of the subject,
placing the device on a body of the subject such that said two electrical contacts of said interdigitated portion are contacting the skin of the subject;
said device measuring the skin capacitance of the subject; and
said device activating the ketosis signal when the capacitance of the skin of the subject has increased compared to the comparative capacitance value of the skin of the subject.

In some embodiments of the invention, the capacitance of the skin has increased by approximately an order of magnitude compared to the comparative capacitance value of the skin of the subject.

As will be appreciated by one of skill in the art, the ketosis signal may be "activated" compared to the state of the ketosis signal when the subject is not in ketosis. That is, if the ketosis signal is in a first condition when the subject is not in ketosis, transitioning the ketosis signal to a second condition "activates" the ketosis signal.

In some embodiments of the invention, there is provided a device for non-invasively detecting if a subject is in ketosis comprising:
an interdigitated portion for measuring capacitance of skin of the subject, said interdigitated portion having at least two separate electrical contacts;
said two electrical contacts of said interdigitated portion arranged for contacting the skin of the subject;
a power source;
a ketosis signal connected to or operably linked to one end of the interdigitated portion, said ketosis signal arranged for activation when the capacitance of the skin of the subject increases compared to a comparative capacitance value, for example, the normal capacitance of the skin of the subject, for example, the capacitance of the skin of the subject when the subject is not in ketosis.

In some embodiments, the device further comprises a capacitance meter.

In another embodiment, the apparatus of the present invention further includes a ketosis exit signal that indicates when the skin capacitance decreases compared to the comparative capacitance value, which is indicative of the degree or rate of fat burning of the user decreasing, the second signal being received from the same interdigitated portion.

In some embodiments, the ketosis exit signal indicates when the skin capacitance is approximately the same as the baseline capacitance value, that is, the ketosis signal indicates when the user has exited ketosis and is no longer burning fat.

In one embodiment, the present invention relates to a method for monitoring fat burning in a subject non-invasively, the method comprising measuring an electrical capacitance of the skin of a subject, wherein when the electric capacitance of the skin is in a nano farad range, the subject is burning fat.

In another embodiment, there is provided a method of monitoring fat burning in a subject, the method comprising taking a first skin capacitance measurement of skin of the subject, after a suitable interval, taking a second capacitance measurement of the skin of the subject, and comparing the second capacitance measurement to the first capacitance measurement, wherein if the second capacitance measurement is greater than the first capacitance measurement, fat burning, that is, the level or degree of fat burning of the subject, has increased during the suitable interval. As will be appreciated by one of skill in the art, in some embodiments of the invention, the step of taking the second capacitance measurement may be repeated, thereby providing a method for monitoring for any substantial or significant or statistically significant increase in capacitance wherein the increase is indicative of an increase in fat burning, that is, the degree or level of fat burning or an increase in the level or degree of ketosis of the subject.

Alternatively, in some embodiments, a frequency sweep is done to determine the frequencies for the strongest capacitance reading. Specifically, in these embodiments, the device determines what frequencies have the biggest difference for example, between a depth reading and the surface reading when the individual is in ketosis. As will be appreciated by one of skill in the art, in some embodiments, more than two frequencies may be used, which would give greater information regarding the acetone gradient, as discussed herein. As discussed above, this information may be stored in memory for subsequent use so that the combination of frequencies can be used again. That is, the combination of frequencies may be stored as preferred frequencies.

In some embodiments, the device is arranged to scan through two or more frequencies from between 0 Hz or DC to about 100 kHz. In some embodiments of the invention, the device scans two or more frequencies selected from the group consisting of about 0 Hz (DC), about 10kHz, about 20 kHz, about 30 kHz, about 40 kHz, about 50 kHz, about 60 kHz, about 70 kHz, about 80 kHz, about 90 kHz and about 100 kHz.

As will be appreciated by one of skill in the art, acetone enters the interstitial fluid from deep within the body and works its way to the surface of the skin where it is evaporated off. Accordingly, acetone production is either increasing or decreasing depending on whether the person is using (burning) more or less fat. Furthermore, concentrations of acetone at different depths may vary compared to the surface concentration if the evaporation rate is higher than the rate of acetone production or not. Both give useful information.

As discussed herein, a significant advantage of measuring capacitance at more than one depth or location is that greater information can be provided, for example, about the degree or intensity of the fat burning or ketosis as well as whether the user is going into or coming out of ketosis. For example, if the depth capacitance reading is greater than the surface capacitance reading, more acetone is moving to the skin surface, indicating that ketosis is increasing. If the surface capacitance reading and the depth capacitance reading are approximately the same, ketosis is in a steady state. If the surface capacitance reading is greater than the depth capacitance reading, ketosis or the rate of ketone production is decreasing. As discussed herein, this provides the user with information on the trend of ketosis so that the user can act accordingly. For example, if ketosis is increasing, the user may decrease the intensity of their workout so as to reach a steady state. Alternatively, if ketosis is decreasing, the individual may increase the intensity of the workout so that more fat is burned.

For example, if ketones are increasing over time, which can be determined either by measuring skin capacitance at one depth over time or by comparing skin capacitance at two or more depths, the person knows that they are "deepening" their degree of ketosis. As another example, a long distance runner may want to target a specific ketone level since fat burning is continuously available during the race and their ketone level will increase during the race. As another example, a person trying to lose weight might feel better at one ketone level than another and may want to target a specific level. As will be apparent to one of skill in the art, these are examples of suitable comparative capacitance values. Furthermore, in these instances, the device may be configured so that the ketosis signal is activated, that is, either turned off or on, whenever the current skin capacitance measurement varies from the comparative capacitance value by a certain degree or amount, for example, ± 25%, ±20%, ±15% or ±10%. Similarly, by taking measurements at one or more depths, the device can report on the trend in capacitance, for example, whether capacitance is changing abruptly, as discussed herein.

As can be seen, depth profiling gives an instantaneous reading of increasing or decreasing acetone levels and this is confirmed by the changes over time.

In another embodiment, there is provided a method of monitoring fat burning in a subject, the method comprising taking a first capacitance measurement at a first skin depth of the subject, and taking a second capacitance measurement at a second skin depth of the subject, and comparing the second capacitance measurement to the first capacitance measurement, wherein if the second capacitance measurement is greater than the first capacitance measurement, fat burning, that is, the level or degree of fat burning of the subject, is increasing if the second depth is greater than the first depth but is decreasing if the first depth is greater than the second depth relative to the skin surface. In some embodiments, either the first skin depth or the second skin depth may be the skin surface. As will be appreciated by one of skill in the art, in some embodiments of the invention, additional capacitance measurements may be taken at different depths, as discussed herein.

In some embodiments, the device is arranged such that the alternating current can be varied over (swept through) a range of frequencies for measuring capacitance at a range of skin depths, as discussed herein.

In some embodiments, the device is arranged to scan through two or more frequencies from between 0 Hz or DC to about 100 kHz. In some embodiments of the invention, the device scans two or more frequencies selected from the group consisting of about 0 Hz (DC), about 10kHz, about 20 kHz, about 30 kHz, about 40 kHz, about 50 kHz, about 60 kHz, about 70 kHz, about 80 kHz, about 90 kHz and about 100 kHz.

As discussed above, capacitance may be measured when the individual is not in ketosis or burning fat as a control or for comparison purposes and may serve as a baseline capacitance value as discussed herein. As will be appreciated by one of skill in the art, this control reading does not necessarily need to be repeated each time the user uses the device, as discussed herein, but may be stored as a baseline capacitance value or measurement. Alternatively, as discussed above, one or more comparative capacitance values or measurements may be stored, each indicative of a different relative state of ketosis.

(Note: Acetone has a CAS Registry No.: 67-64-1 and has these other names: 2-Propanone, Dimethyl ketone, Propanone). Three molecules are grouped together as "ketone bodies", acetoacetate, b-hydroxybutyrate and acetone.

As discussed herein, the device is an apparatus for non-invasively measuring ketosis. Ketosis can be used as an indication that a subject is burning fat. As such, the device is an apparatus for monitoring fat-burning in a subject.

In some embodiments, the apparatus comprises means for measuring the electric capacitance of the skin of a subject, and an out-put signal configured to be indicative of the subject burning fat or not burning fat.

In some embodiments, the apparatus comprises (i) a capacitance meter, (ii) an interdigitated portion having at least two opposing ends and configured for contacting a skin of the subject and for measuring surface capacitance of the subject's skin; (iii) a ketosis signal that changes when the capacitance of the skin is in a range indicative of the subject burning fat, for example, in the nano farad range, the ketosis signal being connected to or responsive to or operably linked to one end of the interdigitated portion and (iv) a power source.

In some embodiments of the invention, the device further comprises an out-put signal that turns on or is activated when the electrical capacitance of the skin of the subject or of the breath of the subject is in a picofarad range, indicative of the subject not burning fat.

In some embodiments of the invention, the power source is an alternating power source. In another embodiment of the invention, the device is arranged such that the alternating current can be varied over (swept through) a range of frequencies to determine the frequency best suited determining if the individual is burning fat, for example, for the individual and for the depth of penetration and interest, as discussed herein.

In another embodiment of the invention, the device further comprises a processor and a memory unit, as discussed herein.

As discussed herein, the movement of acetone to the skin surface results in changes in skin capacitance and this movement of acetone is indicative of fat burning. Typically, this change in capacitance is about two orders of magnitude but will of course depend on the normal skin capacitance of the individual and exactly how much acetone has moved to the skin surface. For example, an individual may have a skin capacitance of about 0.002 µf when not in ketosis but have a skin capacitance of about 0.2 µf when in ketosis or burning fat.

As will be apparent to one of skill in the art, the information provided by the apparatus has several uses. For example, if a person is fasting to get into ketosis, once they are in ketosis, they can start to eat a low glycemic index diet and then they can monitor their degree of ketosis to stay in a safe range. Alternatively, for someone wishing to starve cancer of its primary source of nutrients (glucose) as recommended by Thomas Seyfried in his book "Cancer as a Metabolic Disease" needs to maintain a ketosis level that equates to their lowest glucose level and this could be monitored and maintained with this device.

Furthermore, information on the acetone gradient would allow the user to determine if they are moving towards a different degree of ketosis (for example, mild, medium, nutritional or deep). This is an indication of increasing or decreasing the amount of fat burning so that the user could act accordingly, for example, increasing, decreasing or maintaining the exercise intensity as desired.

It is also important to note that the transition into ketosis happens over a relatively short period of time such as for example 20-30 minutes. It is also important to note that if even if a small amount of glucose is available, the body will preferentially use this glucose as fuel, delaying the use of available ketones until the glucose is no longer available. When glucose is available there is a quick and temporary drop in the level of acetone in the interstitial fluid and this is detected and indicated by the device.

As such, the apparatus can effectively measure the sweat on the skin and the subcutaneous interstitial fluids in the skin of the individual for non-invasively measuring ketosis.

Also disclosed is a method for monitoring fat burning in a subject non-invasively, the method comprising measuring an electrical capacitance of the skin of a subject, wherein if the electrical capacitance of the skin is in nano farad range, the subject is burning fat. As discussed above, electrical capacitance of the skin changes when the subject is burning fat compared to when they are not burning fat.

For example, a comparison of capacitances may be measured by two different oscillators (i.e. depth profiling the changes in capacitance), thereby enabling measurement of the migration of acetone to the skin. In other words, it is possible to measure the gradient of the acetone concentrations as the acetone makes its way to the surface of the skin. For example, the first derivative of any one depth or the comparison of two depths indicates the flow rate and thereby indicates whether acetone levels are increasing or decreasing.

As will be appreciated by one of skill in the art, shorter frequencies of oscillation will obtain measurements that are closer to the skin surface while longer frequencies will obtain measurements from deeper in the tissue.

While this may be done with two different oscillators, in some embodiments, a single oscillator arranged to function at two or more frequencies, for example, a scanning oscillator, may be used in the device, as discussed herein.

Alternatively, a single oscillator may be used for measurements. In these embodiments, measurements may be obtained by varying one or more of the frequencies of oscillation, as discussed herein.

The proximity or spacing of the interdigitated traces, in contact with the user's skin can also be varied to put the measured capacitance in an acceptable range. We have found that spacing difference of about 0,127mm (0,005 inch) are optimal. For example, in some embodiments of the invention, three pairs of interdigitated traces 0,127mm, 01254mm and 0,381mm (0.005, 0,010 and 0,015 inches) apart yield acceptable readings, although other suitable spacings which will generate suitable frequencies will be readily apparent to one of skill in the art and can be determined through routine experimentation and/or optimization.

For example, in some embodiments, one oscillator reads one pair of ends or interdigitated traces while the other oscillator reads a second pair of interdigitated traces. One pair would be in contact with the skin and one very close, but not in contact with the skin. The voltage from each of the oscillators would be sensed between the pairs and would measure the tissue between and under the contact/traces attached to that oscillator. Alternatively, in other embodiments, different frequencies could be placed on the same set of interdigitated traces.

Thus, the device and the method effectively provide a ketosis signal that is indicative of the capacitance of the subcutaneous tissue below the skin changes as the subject's metabolism changes to a state in which fat is being burned, that is, acetone is being produced and/or moved to the surface of the skin.

As discussed herein, subcutaneous capacitance may be measured by a variety of means, for example, by using a temperature-compensated oscillator or comparing two matched oscillators, wherein one is in closer proximity to the skin. As discussed above, the ability to compare, for example, surface to sub-surface capacitance or to compare sub-surface capacitance at two different depths, allows the determination or measurement of a spatial gradient of acetone.

In one particular aspect of the method, the electric capacitance of the skin is measured using one of the above described apparatuses.

The device is an apparatus for non-invasively measuring ketosis. Ketosis can be used as an indication that a subject is burning fat. As such, the device is an apparatus for monitoring fat-burning in a subject.

The apparatus has means for measuring an electric capacitance of the skin or breath of a subject and at least one out-put signal configured to indicate when the electric capacitance of the skin or breath is indicative of the subject burning fat or not burning fat.

In some embodiments, the apparatus comprises a capacitance meter, which includes: (i) an interdigitated portion comprising close proximity conductors (preferably indium tin oxide or a non-oxidizing metallic, although any suitable conductor may be used) and having two opposing ends and the interdigitated portion being configured for contacting a skin of the subject or configured for receiving breath from the subject and measure surface capacitance of the subject's skin; (ii) a ketosis signal that indicates when the capacitance of the skin is indicative of the subject burning fat, the ketosis signal being connected to or responsive to or operably linked to one end of the interdigitated portion and (iii) an alternating power source.

### Ketometer

Alternatively, the apparatus can measure surface or subsurface capacitance with an oscillator, which includes: (i) at least one interdigitated portion comprising close proximity conductors (preferably indium tin oxide or a non-oxidizing, metallic, although any suitable conductor may be used) and having two opposing ends and the interdigitated portion being configured for contacting a skin of the subject or configured for receiving breath from the subject and measure surface conductivity of the subject's skin or the conductivity of the skin; (ii) a ketosis signal that indicates when the capacitance of the skin is in nano farad range indicative of the subject burning fat, the ketosis signal being connected to or operably linked to or responsive to one end of the interdigitated portion and (iii) a power source. In some embodiments, the power source is an alternating power source.

As discussed herein, in these embodiments, this device may have a pair of matched frequency oscillators. The difference between these two gives an indication of the capacitance of the skin while reducing the temperature drift normally experienced. For example, by varying the frequency of the oscillator pair from a few hertz (under 10) to approximately 100 kHz will provide depth profile information about the surface and subsurface capacitance. The comparison between the two readings of the capacitance at two skin depths provides information about an acetone gradient, as discussed above.

In some embodiments, the device is arranged to be worn on the wrist. Readings may be taken on a suitable schedule, for example, every minute or so or at another suitable interval and the readings recorded/stored together with a time stamp. The device would have the ability to extract the readings to review the data and to look for changes in capacitance or ideally indicate significant changes in capacitance by reporting same to the user, for example, by illuminating a series of green LEDs or by reporting to another device such as a smart phone, as discussed herein. That is, in some embodiments, the illumination of LEDs around the outside of the device either in terms of number or brightness may be increased as capacitance increases and conversely reduced when the capacitance drops.

As discussed herein, the method is based largely on an increase in capacitance of approximately two orders of magnitude, not specifically for an absolute value of capacitance, although suitable values are provided as a guide for one of skill in the art.

In some embodiments, the device may be arranged with the ability to initially null the frequency difference between the two oscillators after installation on the wrist. As will be appreciated by one of skill in the art, if you don't null the two you would need to know the difference and watch for increases or decreases in that difference.

As will be appreciated by one of skill in the art, temperature drift will be the same for both oscillators and should have a minimal influence on the readings.

We are primarily concerned with repeat-ability within any one user.

In some embodiments, data is used to develop a curve to map/identify the capacitance changes as experience and/or data is gained about when and how the person goes in and out of ketosis.

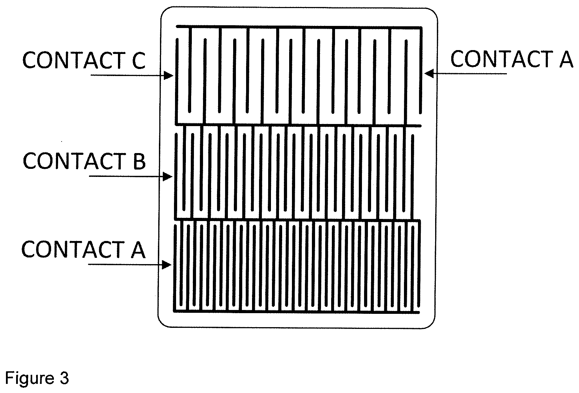

In some embodiments, a secondary capacitance comparison is made using an AC (for example, 8 Hz) differently spaced interdigitated surface contact. This would additionally take advantage of the surface acoustic wave properties (SAW) since these are also influenced by changes to the mechanical properties of the tissue are influenced by acetone concentrations and these will change as acetone levels build up. In some embodiments, this would be three differently spaced interdigitated contact sensors as shown in Figure 3.

As can be seen in Figure 3, each conductive strip has traces that pass between the other two strips. The proximity of the strips allows for accurate measurements of the capacitance between the adjacent strips and allows for comparisons between the relative spacings. It is further of note that in those embodiments wherein contact capacitance is measured, the conductive strips may not be coated; however, in those embodiments wherein oscillators are used, the conductive strips may be coated.

The apparatus can measure the sweat on the skin of the individual or in the breath of the individual for non-invasively measuring ketosis.

Also disclosed is a method for monitoring fat burning in a subject non-invasively, the method including the steps of measuring an electric capacitance of the skin or breath of a subject, wherein when the electrical capacitance of the skin or breath is in a range that is indicative of the subject burning fat.

**In** one particular aspect of the method, the electric capacitance of the skin is measured using one of the above described apparatuses.

The device is an apparatus for non-invasively detecting whether a person is in a state of ketosis. Ketosis can be used as an indication that the subject is burning fat. As such, the device is an apparatus for monitoring fat-burning in a subject.

The apparatus has means for measuring an electrical capacitance of the skin or breath of a subject, and at least one out-put signal configured to indicate when the electrical capacitance of the skin or breath is in a nano farad range indicative of the subject burning fat and another (optional) out-put signal that turns on when the electrical capacitance of the skin or breath of the subject is in a pico farad range, indicative of the subject not burning fat.

### The Device

In accordance with one embodiment, the device uses skin capacitance to differentiate between ranges of capacitance as a test for ketosis.

A measurement of the surface capacitance can be made with a capacitance meter; however, special provisions must be made to adapt a comparative instrument, to allow it to detect the desired capacitances.

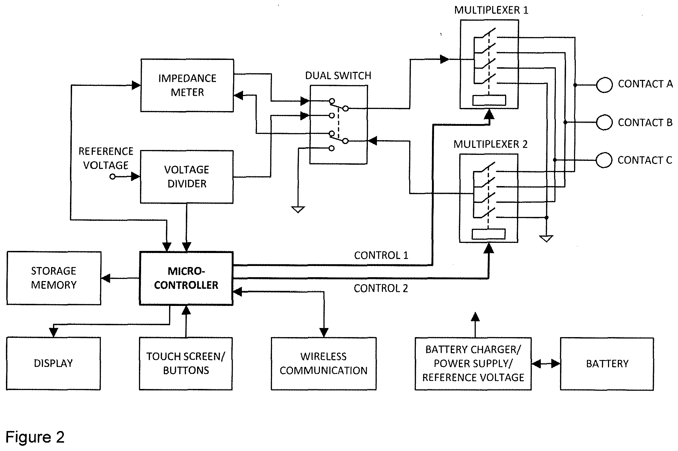

Fig. 2 illustrates an embodiment of a circuit diagram of the capacitance meter of the present invention.

With reference to Fig. 2, electrical contacts (P) section of the capacitance meter 10 where the skin capacitances are to be measured, are fitted preferably with an indium tin oxide (ITO) coated plastic skin contact sheet. The P section may also be referred to as the interdigitated area. The plastic sheet is etched to form an interdigitated labyrinth of the appropriate spatial frequency to achieve the desired capacitance ranges such that the relatively low capacitance measured during normal metabolic states can be differentiated from the higher capacitive states associated with being in ketosis.

The general skin capacitance of individuals may vary. Varying the spatial frequency of the interdigitated contact area changes the capacitance range and allows the device to be selectively matched to users with different base line capacitances. In one embodiment, the spatial frequency of the interdigitated area is approximately 1, 2, and 3 per mm. In another embodiment, the spatial frequency of the interdigitated area is equal or greater than 1 per mm.

The contact area may also be interdigitated, electrically separated, stainless (or other non-corroding, coated conductor) wires. ITO coated plastic skin contact sheet is preferred because of its affinity to acetone. The interdigitated contact area may be made of stainless steel or individual wires coated to prevent corrosion, or an exposed coated printed circuit board or foil. In another embodiment the contact area may be a single use contact surface. This would eliminate any complications that may arise due to surface cleaning or degradation of the contact area.

A power supply section of the meter may be of any suitable type and is illustrated in an alternating current (AC) signal, or equivalent. In another embodiment the supplied voltage is a variable alternating current ranging from DC or zero or a few hertz to the high M Hz range. This concentrates the current flow on the surface of the skin to further differentiate skin capacitance from subcutaneous capacitance.

In another embodiment of the present invention, the device includes both low frequency and high frequency AC. In this embodiment the current flow difference measured across the contact area using both low frequency and high frequency AC is compared. This contrasts the contributions to capacitance that comes from the subcutaneous path to that of the surface capacitance.

The capacitance meter 10 is operative when the electrical contact section 12 is in contact with a subject's skin.

The device of the present invention, in another embodiment, includes processing means and it may also include a memory module and a transmitter to communicate with a smart phone for display of the ketosis indication, as discussed herein.

The processing means may be a programmable processor included to execute program instructions to guide a data process module to directly store captured data into the memory module, to initiate data upload from the memory module to a computing means via data upload module, to compare current readings to initial reading, to manage energy usage and so forth.

The memory module may include any appropriate type of memory now known or later developed including without limitation, read-only memory (ROM), random access memory (RAM), flash memory, and a set of registers included within the programmable processor.

In one embodiment, the processing means may take the form of a microprocessor such as the Maxim Health Sensor Platform MASXREFDES 100#. Maxim MAXREFDES 100# health sensor platform is an integrated sensor platform that helps customers evaluate Maxim's complex and innovative medical and high-end fitness solutions. The platform integrates one biopotential analog front-end solution (MAX30003), one pulse oximeter and heart-rate sensor (MAX30101), two human body temperature sensors (MAX30205), one 3-axis accelerometer, one 3D accelerometer and 3D gyroscope, and one absolute barometric pressure sensor. As will be apparent to one of skill in the art, in other embodiments, other suitable processing means may also be used.

In another embodiment, the device of the present invention may include wireless communication capabilities that would allow it to connect with a computing device, such as a laptop, a desktop, wireless devices such as cellular phones and pads, or to directly connect to the Internet. The information stored in the memory means may be downloaded into a computing device for storage or for further analysis.

If the person is following a program to achieve ketosis such as fasting or dieting, the indicator informs the user whether they are in a fat burning state or not.

The device of the present invention may be used on any part of the body. The top of the fingertip is a site particularly well suited for performing skin conductivity. The inside of the forearm is yet another particularly well-suited part for performing skin conductivity with the device of the present invention. The ear lobe is yet another site particularly well suited for performing skin conductivity. As another example, we have found that the underside of the wrist is a good location for the contact sensor.

Without further elaboration, it is believed that one of ordinary skill in the art can, based on the description presented herein, utilize the present invention to the full extent.

Future applications claiming priority to this application may or may not include the following claims, and may include claims broader, narrower, or entirely different from the following claims.

The scope of the claims should not be limited by the preferred embodiments set forth in the examples but should be given the broadest interpretation consistent with the description as a whole.

## Claims

1. A device comprising:
(i) an interdigitated portion having two opposing ends and configured for contacting skin of a subject and measuring skin capacitance;
(ii) a ketosis signal , and
(iii) a capacitance meter;
**characterized in that** the ketosis signal is activated when the skin capacitance measurement is in the nano farad range.

2. The device according to claim 1 wherein the interdigitated portion is arranged to measure the skin capacitance at a skin surface.

3. The device according to claim 1 wherein the device is arranged to measure the skin capacitance at one or more skin depths, for example, by comprising interdigitated traces spaced 0,127mm, 01254mm and 0,381mm (0.005, 0,010 and 0,015 inches) apart.

4. The device according to claim 3 wherein the device is arranged to scan through two or more frequencies from between 0 Hz or DC to about 100 kHz, for example, at least two or more frequencies selected from the group consisting of about 0 Hz (DC), about 10kHz, about 20 kHz, about 30 kHz, about 40 kHz, about 50 kHz, about 60 kHz, about 70 kHz, about 80 kHz, about 90 kHz and about 100 kHz.

5. The device according to any one of claims 1-4 wherein the interdigitated portion is also configured to measure skin resistance.

6. The device according to claim 1 wherein the device further comprises a ketosis exit signal arranged to activate when the skin capacitance measurement is in the pico farad range.

7. A method for non-invasively detecting if a subject is in ketosis comprising:
providing a device comprising:
an interdigitated portion for measuring capacitance of skin of the subject, said interdigitated portion having at least two separate electrical contacts;
and
a ketosis signal responsive to one end of the interdigitated portion, said ketosis signal being activated when a skin capacitance measurement of the subject increases compared to a comparative capacitance value of the subject,
placing the device on a body of the subject such that said two electrical contacts of said interdigitated portion are contacting the skin of the subject; and
said device measuring the capacitance of the skin of the subject;
**characterized in that** said device activates the ketosis signal when the skin capacitance measurement is different from the comparative capacitance measurement of the subject.

8. The method according to claim 7 wherein the comparative capacitance value is a baseline capacitance measurement corresponding to skin capacitance when not in ketosis, for example, in the pico farad range.

9. The method according to claim 8 wherein the ketosis signal is activated when the skin capacitance measurement is greater than the baseline capacitance measurement, for example, at least one order of magnitude greater than the baseline capacitance measurement, preferably at least two orders of magnitude greater than the baseline capacitance measurement.

10. The method according to claim 7 wherein the interdigitated portion is arranged to measure the skin capacitance at a skin surface.

11. The method according to claim 7 wherein the device is arranged to measure the skin capacitance at one or more skin depths, for example, by comprising interdigitated traces spaced 0,127mm, 01254mm and 0,381mm (0.005, 0,010 and 0,015 inches) apart..

12. The device according to claim 11 wherein the device is arranged to scan through two or more frequencies from between 0 Hz or DC to about 100 kHz, for example, at least two or more frequencies selected from the group consisting of about 0 Hz (DC), about 10kHz, about 20 kHz, about 30 kHz, about 40 kHz, about 50 kHz, about 60 kHz, about 70 kHz, about 80 kHz, about 90 kHz and about 100 kHz.

13. The device according to any one of claims 7-12 wherein the interdigitated portion is also configured to measure skin resistance, for example, wherein the capacitance measurement is adjusted based on a resistance measurement prior to comparison with the comparative capacitance value.

14. The method according to claim 7 wherein the ketosis signal is activated whenever the skin capacitance measurement varies from the comparative capacitance value by 10%.

15. The method according to any one of claims 7-14 wherein the device further comprises a ketosis exit signal arranged to activate when the skin capacitance measurement is approximately the baseline capacitance value of the skin of the subject.

16. The method according to claim 7 wherein measuring the capacitance of the skin comprises:
measuring a first capacitance at a first skin depth of the subject, and
measuring a second capacitance at a second skin depth of the subject.

17. The method according to claim 16 wherein if the second capacitance at the second skin depth is greater than the first capacitance at the first skin depth, a degree of ketosis of the subject is increasing if the second depth is greater relative to the skin surface than the first depth but the degree of ketosis of the subject is decreasing if the first depth is greater than the second depth relative to the skin surface.

## Patentansprüche

1. Vorrichtung, umfassend:
(i) einen interdigitierten Abschnitt mit zwei gegenüberliegenden Enden, der für den Kontakt mit der Haut eines Probanden und die Messung der Hautkapazität konfiguriert ist;
(ii) ein Ketosesignal und
(iii) ein Kapazitätsmessgerät;
**dadurch gekennzeichnet, dass** das Ketosesignal aktiviert wird, wenn die Hautkapazitätsmessung im Nanofaradbereich liegt.

2. Vorrichtung nach Anspruch 1, wobei der interdigitierte Abschnitt dazu dient, die Hautkapazität an einer Hautoberfläche zu messen.

3. Vorrichtung nach Anspruch 1, wobei die Vorrichtung dazu ausgelegt ist, die Hautkapazität in einer oder mehreren Hauttiefen zu messen, beispielsweise indem sie ineinandergreifende Spuren umfasst, die 0,127 mm, 01254 mm und 0,381 mm (0,005, 0,010 und 0,015 Zoll) voneinander beabstandet sind.

4. Vorrichtung nach Anspruch 3, wobei die Vorrichtung dazu eingerichtet ist, zwei oder mehr Frequenzen zwischen 0 Hz oder DC und etwa 100 kHz, beispielsweise mindestens zwei oder mehr Frequenzen abzutasten, die aus der Gruppe ausgewählt sind, die aus etwa 0 Hz (DC), etwa 10 kHz, etwa 20 kHz, etwa 30 kHz, etwa 40 kHz, etwa 50 kHz, etwa 60 kHz, etwa 70 kHz, etwa 80 kHz, etwa 90 kHz und etwa 100 kHz besteht.

5. Vorrichtung nach einem der Ansprüche 1 bis 4, wobei der interdigitierte Abschnitt auch zur Messung des Hautwiderstands konfiguriert ist.

6. Vorrichtung nach Anspruch 1, wobei die Vorrichtung ferner ein Ketose-Ausgangssignal umfasst, das aktiviert wird, wenn die Hautkapazitätsmessung im Picofarad-Bereich liegt.

7. Verfahren zum nicht-invasiven Feststellen, ob sich ein Proband in Ketose befindet, umfassend:
Bereitstellen einer Vorrichtung, umfassend:
einen interdigitalen Abschnitt zum Messen der Kapazität der Haut des Probanden, wobei der interdigitale Abschnitt mindestens zwei separate elektrische Kontakte aufweist;
und
ein Ketosesignal, das auf ein Ende des interdigitierten Abschnitts reagiert. Das Ketosesignal wird aktiviert, wenn ein Hautkapazitätswert des Probanden im Vergleich zu einem vergleichbaren Kapazitätswert des Probanden ansteigt,
Platzieren der Vorrichtung auf einem Körper des Probanden, so dass die beiden elektrischen Kontakte des interdigitierten Abschnitts die Haut des Probanden berühren; und
wobei die Vorrichtung die Kapazität der Haut des Probanden misst;
**dadurch gekennzeichnet, dass** die Vorrichtung das Ketosesignal aktiviert, wenn die Hautkapazitätsmessung von der Vergleichskapazitätsmessung des Probanden abweicht.

8. Verfahren nach Anspruch 7, wobei der Vergleichskapazitätswert eine Basiskapazitätsmessung ist, die der Hautkapazität außerhalb der Ketose entspricht, beispielsweise im Picofarad-Bereich.

9. Verfahren nach Anspruch 8, wobei das Ketosesignal aktiviert wird, wenn der Hautkapazitätsmesswert größer ist als der Basiskapazitätsmesswert, beispielsweise mindestens eine Größenordnung größer als der Basiskapazitätsmesswert, vorzugsweise mindestens zwei Größenordnungen größer als der Basiskapazitätsmesswert.

10. Verfahren nach Anspruch 7, wobei der interdigitierte Abschnitt dazu dient, die Hautkapazität an einer Hautoberfläche zu messen.

11. Verfahren nach Anspruch 7, wobei die Vorrichtung so beschaffen ist, dass sie die Hautkapazität in einer oder mehreren Hauttiefen misst, beispielsweise indem sie ineinander greifende Spuren mit einem Abstand von 0,127 mm, 01254 mm und 0,381 mm (0,005, 0,010 und 0,015 Zoll) aufweist.

12. Vorrichtung nach Anspruch 11, wobei die Vorrichtung dazu eingerichtet ist, zwei oder mehr Frequenzen zwischen 0 Hz oder DC und etwa 100 kHz, beispielsweise mindestens zwei oder mehr Frequenzen abzutasten, die aus der Gruppe ausgewählt sind, die aus etwa 0 Hz (DC), etwa 10 kHz, etwa 20 kHz, etwa 30 kHz, etwa 40 kHz, etwa 50 kHz, etwa 60 kHz, etwa 70 kHz, etwa 80 kHz, etwa 90 kHz und etwa 100 kHz besteht.

13. Vorrichtung nach einem der Ansprüche 7 bis 12, wobei der interdigitierte Abschnitt auch dazu konfiguriert ist, beispielsweise den Hautwiderstand zu messen, wobei die Kapazitätsmessung vor dem Vergleich mit dem vergleichbaren Kapazitätswert auf der Grundlage einer Widerstandsmessung angepasst wird.

14. Verfahren nach Anspruch 7, wobei das Ketosesignal immer dann aktiviert wird, wenn die Hautkapazitätsmessung um 10 % vom Vergleichskapazitätswert abweicht.

15. Verfahren nach einem der Ansprüche 7 bis 14, wobei die Vorrichtung ferner ein Ketose-Ausgangssignal umfasst, das aktiviert wird, wenn die Hautkapazitätsmessung etwa dem Basiskapazitätswert der Haut des Probanden entspricht.

16. Verfahren nach Anspruch 7, wobei das Messen der Hautkapazität Folgendes umfasst:
Messen einer ersten Kapazität in einer ersten Hauttiefe des Probanden und
Messen einer zweiten Kapazität in einer zweiten Hauttiefe des Probanden.

17. Verfahren nach Anspruch 16, wobei, wenn die zweite Kapazität bei der zweiten Hauttiefe größer ist als die erste Kapazität bei der ersten Hauttiefe, der Grad der Ketose des Probanden zunimmt, wenn die zweite Tiefe im Verhältnis zur Hautoberfläche größer ist als die erste Tiefe, der Grad der Ketose des Probanden jedoch abnimmt, wenn die erste Tiefe im Verhältnis zur Hautoberfläche größer ist als die zweite Tiefe.

## Revendications

1. Dispositif comprenant :
(i) une partie interdigitée ayant deux extrémités opposées et configurée pour entrer en contact avec la peau d'un sujet et mesurer la capacité cutanée ;
(ii) un signal de cétose, et
(iii) un capacimètre ;
**caractérisé en ce que** le signal de cétose est activé lorsque la mesure de la capacité cutanée est de l'ordre du nanofarad.

2. Dispositif selon la revendication 1, dans lequel la partie interdigitée est agencée pour mesurer la capacité cutanée à une surface cutanée.

3. Dispositif selon la revendication 1, dans lequel le dispositif est agencé pour mesurer la capacité cutanée à une ou plusieurs profondeurs cutanées, par exemple, en comprenant des traces interdigitées espacées de 0,127 mm, 0,1254 mm et 0,381 mm (0,005, 0,010 et 0,015 pouce).

4. Dispositif selon la revendication 3, dans lequel le dispositif est agencé pour balayer deux ou plusieurs fréquences entre 0 Hz ou CC et environ 100 kHz, par exemple, au moins deux ou plusieurs fréquences sélectionnées dans le groupe constitué d'environ 0 Hz (CC), d'environ 10 kHz, d'environ 20 kHz, d'environ 30 kHz, d'environ 40 kHz, d'environ 50 kHz, d'environ 60 kHz, d'environ 70 kHz, d'environ 80 kHz, d'environ 90 kHz et d'environ 100 kHz.

5. Dispositif selon l'une quelconque des revendications 1 à 4, dans lequel la partie interdigitée est aussi configurée pour mesurer la résistance cutanée.

6. Dispositif selon la revendication 1, dans lequel le dispositif comprend également un signal de sortie de cétose agencé pour s'activer lorsque la mesure de capacité cutanée est de l'ordre du picofarad.

7. Procédé de détection non invasive de l'état de cétose d'un sujet, comprenant :
la fourniture d'un dispositif comprenant :
une partie interdigitée pour mesurer la capacité cutanée du sujet, ladite partie interdigitée ayant au moins deux contacts électriques séparés ;
et
un signal de cétose sensible à une extrémité de la partie interdigitée, ledit signal de cétose étant activé lorsqu'une mesure de capacité cutanée du sujet augmente par rapport à une valeur de capacité comparative du sujet,
le placement du dispositif sur le corps du sujet de telle sorte que les deux contacts électriques de ladite partie interdigitée soient en contact avec la peau du sujet ; et
ledit dispositif mesurant la capacité cutanée du sujet ;
**caractérisé en ce que** ledit dispositif active le signal de cétose lorsque la mesure de capacité cutanée est différente de la mesure de capacité comparative du sujet.

8. Procédé selon la revendication 7, dans lequel la valeur de capacité comparative est une mesure de capacité de base correspondant à la capacité cutanée lorsqu'elle n'est pas en cétose, par exemple, de l'ordre du picofarad.

9. Procédé selon la revendication 8, dans lequel le signal de cétose est activé lorsque la mesure de capacité cutanée est supérieure à la mesure de capacité de base, par exemple, au moins un ordre de grandeur supérieur à la mesure de capacité de base, de préférence au moins deux ordres de grandeur supérieurs à la mesure de capacité de base.

10. Procédé selon la revendication 7, dans lequel la partie interdigitée est agencée pour mesurer la capacité cutanée à une surface cutanée.

11. Procédé selon la revendication 7, dans lequel le dispositif est agencé pour mesurer la capacité cutanée à une ou plusieurs profondeurs cutanées, par exemple, en comprenant des traces interdigitées espacées de 0,127 mm, 0,1254 mm et 0,381 mm (0,005, 0,010 et 0,015 pouce).

12. Dispositif selon la revendication 11, dans lequel le dispositif est agencé pour balayer deux ou plusieurs fréquences entre 0 Hz ou CC et environ 100 kHz, par exemple, au moins deux ou plusieurs fréquences sélectionnées dans le groupe constitué d'environ 0 Hz (CC), d'environ 10 kHz, d'environ 20 kHz, d'environ 30 kHz, d'environ 40 kHz, d'environ 50 kHz, d'environ 60 kHz, d'environ 70 kHz, d'environ 80 kHz, d'environ 90 kHz et d'environ 100 kHz.

13. Dispositif selon l'une quelconque des revendications 7 à 12, dans lequel la partie interdigitée est également configurée pour mesurer la résistance cutanée, par exemple, dans lequel la mesure de capacité est ajustée sur la base d'une mesure de résistance avant la comparaison avec la valeur de capacité comparative.

14. Procédé selon la revendication 7, dans lequel le signal de cétose est activé chaque fois que la mesure de capacité cutanée varie de 10 % par rapport à la valeur de capacité comparative.

15. Procédé selon l'une quelconque des revendications 7 à 14, dans lequel le dispositif comprend également un signal de sortie de cétose agencé pour s'activer lorsque la mesure de capacité cutanée est approximativement la valeur de capacité cutanée de base du sujet.

16. Procédé selon la revendication 7, dans lequel la mesure de la capacité cutanée comprend :
la mesure d'une première capacité à une première profondeur cutanée du sujet, et
la mesure d'une seconde capacité à une seconde profondeur cutanée du sujet.

17. Procédé selon la revendication 16, dans lequel si la seconde capacité à la seconde profondeur cutanée est supérieure à la première capacité à la première profondeur cutanée, un degré de cétose du sujet augmente si la seconde profondeur est supérieure par rapport à la surface cutanée à la première profondeur, mais le degré de cétose du sujet diminue si la première profondeur est supérieure à la seconde profondeur par rapport à la surface cutanée.
